# EUROPEAN PATENT APPLICATION

(11) **EP 4 241 777 A1**
(43) Date of publication of application: **13.09.2023**
(21) Application number: 21889575.3
(22) Date of filing: 04.11.2021
(51) Int. Cl.: A61K 35/30, A61P 25/28, C12N 5/0793, C12N 5/00, A61K 35/545

(54) **PHARMACEUTICAL COMPOSITION FOR TREATING DEGENERATIVE BRAIN DISEASE, INCLUDING NEURAL PRECURSOR CELLS DERIVED FROM PLURIPOTENT STEM CELLS**

(30) Priority: 06.11.2020 KR 20200147335
(71) Applicant: SBIOMEDICS, Seoul 04797 (KR)
(72) Inventor: KIM, Minyoung, Seoul 06667 (KR); CHOUNG, Jinseung, Pyeongtaek-si, Gyeonggi-do 17881 (KR); HWANG, Dong-Youn, Yongin-si, Gyeonggi-do 16802 (KR); KIM, Hyun-Mun, Seongnam-si, Gyeonggi-do 13364 (KR)
(74) Representative: Morabito, Sara
(86) International application number: PCT/KR2021/015866
(87) International publication number: WO 2022/098110

(57) **Abstract**

Provided is a pharmaceutical composition for treating a degenerative brain disease, comprising pluripotent stem cells-derived neural precursor cells. By injecting the neural precursor cells differentiated from pluripotent stem cells to an amyloid beta-injected animal model through an intracerebroventricular route, the effect of strengthening cognitive function can be sustained for a long period of time, and thus, the neural precursor cells may be effectively used for treatment of degenerative brain diseases including Alzheimer's disease.

## Description

### Technical Field

The present disclosure relates to a pharmaceutical composition for treating a degenerative brain disease including neural precursor cells derived from pluripotent stem cells.

### Background Art

Degenerative brain disease refers to a disease that causes various symptoms such as impairment of motor and sensory function and impairment of higher-order causal functions such as memory, learning, and computational reasoning due to degenerative changes occurring in neural cells of the central nervous system. Alzheimer's disease, Parkinson's disease, and memory impairment are representative diseases thereof.

Alzheimer's disease refers to various complex cognitive disorders such as degeneration of cognitive function accompanied by memory loss, personality change, and behavioral abnormality and causes irreversible dysfunction such as neuronal death resulting in permanent brain damage. The pathogenic mechanism of Alzheimer's disease is not completely verified, candidates for treatment of dementia caused by amyloid beta have been suggested but mostly failed in clinical trials, and a variety of studies have extensively been conducted on targeting a substance such as tau protein for treatment of dementia. However, even after removing neurofibrillary tangles generated by amyloid beta deposits and tau protein from patients with Alzheimer's disease, effects such as neuro regeneration may not be expected, and thus fundamental treatment via complete neuro regeneration is required. Accordingly, therapeutic agents for dementia by using stem cells have been developed (International Patent Publication No. WO2016088930A1). However, one of the major problems of cell therapeutics is a low viability of transplanted cells (less than 5%), and it is reported that the transplanted cells undergo significant apoptosis immediately after being injected into the body. However, studies on an effective administration route capable of maintaining a long-term therapeutic effect without causing apoptosis of transplanted cells are still insufficient.

### Disclosure

### Technical problem

Provided is a pharmaceutical composition for preventing or treating a degenerative brain disease including neural precursor cells differentiated from pluripotent stem cells as an active ingredient.

Provided is a use of neural precursor cells differentiated from pluripotent stem cells for preparation of a pharmaceutical composition for preventing or treating a degenerative brain disease.

Provided is a method of preventing or treating a degenerative brain disease, the method comprising administering to an individual in need thereof an effective amount of neural precursor cells differentiated from pluripotent stem cells.

### Technical Solution

According to an aspect of the disclosure, a pharmaceutical composition for preventing or treating a degenerative brain disease includes neural precursor cells differentiated from pluripotent stem cells as an active ingredient.

As used herein, the term "pluripotent stem cell" refers to a cell having self-regenerating capacity (the ability of passing through a number of cell division cycles while maintaining an undifferentiated state thereof) and a cell having a multi-differentiation potential into one or more types of cells (the ability to differentiate into one or more types of specialized cells). The pluripotent stem cells may be nuclear transfer pluripotent stem cells (NT-hPSCs), parthenote-derived pluripotent stem cells (pn-hPSCs), induced pluripotent stem cells (iPSCs), and embryonic stem cells (ESCs). In an embodiment, the pluripotent stem cells may be embryonic stem cells (ESCs), and the embryonic stem cells may refer to cells obtained by extracting inner cell mass from an embryo in the blastocyst stage before a fertilized egg implants in a womb of a mother and culturing the extracted cells *in vitro.*

The pluripotent stem cells may be derived from mammals, e.g., humans, mice, rats, apes, cows, horses, pigs, dogs, sheep, goats, or cats.

As used herein, the term "neural precursor cell (NPC)" may be a cell having the capacity for self-renewal and the ability to differentiate into cells of the nervous system. The neural precursor cell may also be referred to as neural progenitor cell (NPC) and neural stem cell (NSC). The neural precursor cells may be differentiated into neurons, astrocytes, or oligodendrocyte. The neural precursor cell may be a cell expressing SOX1.

As used herein, the term "differentiation" refers to a phenomenon in which a structure or function of a cell is specialized while the cell grows by division and proliferation. The pluripotent stem cells may be cells completely differentiated into specific cells after passing through a specific type of neural precursor cells. The embryonic stem cells may be differentiated into the neural precursor cells, and the neural precursor cells may be differentiated into neurons, astrocytes, or oligodendrocytes.

As used herein, the term "degenerative brain disease" may refer to a disease caused by degeneration of a structure or function of brain tissue or brain cells as aging progresses.

In an embodiment, the degenerative brain disease may be a disease selected from the group consisting of Alzheimer's disease, Parkinson's disease, Huntington's disease, mild cognitive impairment, cerebral amyloid angiopathy, Down's syndrome, amyloidotic stroke, systematic amyloidosis, Dutch-type amyloidosis, Niemann-Pick disease, senile dementia, amyotrophic lateral sclerosis, spinocerebellar atrophy, Tourette's syndrome, Friedrich's Ataxia, Machado-Joseph's disease, Lewy body dementia, dystonia, progressive supranuclear palsy, and frontotemporal dementia.

The term "Alzheimer's disease" may be used interchangeably with senile dementia and may refer a disease involving mental degradation associated with certain degenerative brain diseases characterized by senile plaque, neuroinflammatory tangles, and progressive nerve loss.

The composition may be parenterally administered. In an embodiment, the composition may be administered via an intravenous (IV), intrahippocampal (IH), intracerebral, intracranial, intraspinal, intracerebrospinal, intracerebroventricular (ICV), or intrathecal route. The terms "administer", "introduce", "inject" and "transplant" may be used interchangeably and may refer to placement of the composition according to an embodiment in an individual via a method or route leading to at least partial localization of the composition into a desired region. The composition according to an embodiment may be administered via any route suitable for delivery to a desired region in an individual in which a cell or at least a part of the cell is viable. In an embodiment, the composition may be injected via an intracerebroventricular or intrathecal route to reach the cerebrospinal fluid. According to most of existing administration routes used in cell therapeutics, including direct injection into the brain and intravenous injection, significant apoptosis occurs within several days immediately after injection into living bodies. However, according to intracerebroventricular or intrathecal administration of the composition, the survival of cells is maintained for a long time, thereby prolonging cognitive function strengthening effects for a long period of time.

In an embodiment, the neural precursor cells may be differentiated by two-dimensionally culturing an embryonic body (EB) obtained by three-dimensionally culturing the pluripotent stem cells in a culture medium including a protein kinase C-β (PKC-β) inhibitor and a bone morphogenetic protein (BMP) inhibitor.

In an embodiment, the pluripotent stem cells may be obtained by culturing under feeder cell-free conditions.

As used herein, the term "protein kinase C (PKC)" may refer to an enzyme involved in phosphorylation of a protein which regulates functions of the protein by phosphorylating a hydroxyl group of serine and threonine of the protein.

The "PKC-β inhibitor" may inhibits expression or activity of the PKC-β. In an embodiment, the PKC-β inhibitor may be selected from 2-[1-(3-dimethylaminopropyl)-5-methoxy indol-3-yl]-3-(1H-indol-3-yl) maleimide; 3-(1-(3-imidazol-1-yl propyl)-1H-indol-3-yl)-4-anilino-1H-pyrrole-2,5-dione; 3-(1H-indol-3-yl)-4-[2-(4-methylpiperazin-1-yl)quinazolin-4-yl]pyrrole-2,5-dione; (3-{1-[3-(amidinothio)propyl]-1H-indol-3-yl}-3-(1-methyl-1H-indol-3-yl)maleimide methane sulfonate; 13-hydroxyoctadecadienoic acid; bisindolylmaleimide; 2,6-diamino-N-([1-oxotridecyl)-2-piperidinyl]methyl)hexanamide; 4'-demethylamino-4'-hydroxystaurosporine; and 3-(13-methyl-5-oxo-6,7-dihydro-5H-indolo[2,3-a] pyrrolo[3,4-c] carbazol-12(13H)-yl)propanenitrile. The PKC-β inhibitor may be included in a concentration of 5 µM to 25 µM, 5 µM to 20 µM, 6 µM to 18 µM, 8 µM to 15 µM, or 8 µM to 12 µM.

As used herein, the term "bone morphogenetic protein (BMP)" may refer to a growth factor inducing formation of bones and cartilages.

The "BMP inhibitor" may be a low-molecular weight compound or polypeptide that inhibits signal transfer of the BMP. In an embodiment, the BMP inhibitor may be selected from dorsomorphin (6-[4-[2-(1-piperinyl)ethoxy]phenyl]-3-(4-pyridinyl)-pyrazolo[1,5-a]pyrimidine)); dorsomorphin homolog 1 (DMH1, 4-[6-[4-(1-methylethoxy)phenyl]pyrazolo[1,5-a]pyrimidine-3-yl]-quinoline); K 02288 (3-[(6-amino-5-(3,4,5-trimethoxyphenyl)-3-pyridinyl]phenol); LDN 212854 (5-(6-(4-(1-piperazinyl)phenyl)pyrazolo[1,5-a]pyrimidine-3-yl)quinolone); and Noggin polypeptide, and may be, for example, DMH1. The BMP inhibitor may be contained in a concentration of 5 µM to 25 µM, 5 µM to 20 µM, 6 µM to 18 µM, 8 µM to 15 µM, or 8 µM to 12 µM.

In an embodiment, the three-dimensional culture may be performed for 1 day to 10 days, for example, for 2 days to 6 days or 3 days to 5 days to induced differentiation into neuroectoderm.

In an embodiment, the two-dimensional culture may be performed for 1 day to 15 days, for example, for 2 days to 8 days, 3 days to 7 days, or 4 days to 6 days to form a rosette structure.

As used herein, the term "prevention" refers to all actions intended to inhibit or delay development of a degenerative brain disease by administering the pharmaceutical composition.

As used herein, the term "treatment" refers to all actions to alleviate, inhibit the progress of, or prevent a disease, disorder, or condition, or at least one symptom thereof, and the term "active ingredient" or "a pharmaceutically effective dose" refers to an amount of a composition used while performing a process according to the present disclosure to alleviate, inhibit the progress of, or prevent the disease, disorder, or condition, or at least one symptom thereof.

The pharmaceutical composition according to an embodiment may include a pharmaceutically acceptable carrier and/or additive. For example, the pharmaceutical composition may include sterile water, physiological saline, a common buffer (phosphoric acid, citric acid, and other organic acids), a stabilizer, a salt, an antioxidant (ascorbic acid, etc.), a surfactant, a suspension, an isotonic agent, a preservative, or the like. In the case where the pharmaceutical composition is prepared in a formulation suitable for injection, the neural precursor cells may be dissolved in a pharmaceutically acceptable carrier or may be frozen in a solution state in which the neural precursor cells are dissolved.

A dosage of the pharmaceutical composition according to an embodiment may be 1.0 × 10³ to 1.0 × 10¹⁰ cells/kg(body weight) or individual, or 1.0 × 10⁷ to 1.0 × 10⁸ cells/kg(body weight) or based on the neural precursor cells. However, the dosage may be prescribed in various manners depending on factors such as formulation method, administration mode, age, body weight, gender of a patient, severity of disease, diet, administration time, administration route, excretion rate, and drug sensitivity and those skilled in the art may appropriately adjust the dosage in consideration of these factors. Administration frequency may be once or twice or more within the clinically allowable range of side effects, and administration may be applied to one site or two or more sites. The dosage per kg or per individual for non-human animals may be the same as that for humans, or may be converted from the above-described dosage, for example, based on a volume ratio (e.g., average value) between organs (heart, etc.) of the human and animal subjects. Animals to be treated according to an embodiment may be exemplified by humans and other desired mammals, and specifically, may include humans, monkeys, mice, rats, rabbits, sheep, cows, dogs, horses, pigs, and the like.

The pharmaceutical composition according to an embodiment may be formulated in a unit dosage form or into a multidose container using a pharmaceutically acceptable carrier and/or excipient according to a method that may be easily carried out by those skilled in the art to which the present disclosure pertains. In this regard, the formulation may be in a form of a solution, suspension, or emulsion in an oily or aqueous medium, powder, granules, tablets, or capsules. Also, the cell therapeutics may be formulated into an injectable form. In this case, any known components for formulation may be used and the cell therapeutics may be formulated using any method well known in the art.

In an embodiment, a method of differentiating of the pluripotent stem cells into neural precursor cells may include culturing isolated pluripotent stem cells under feeder cell-free conditions; three-dimensionally culturing the pluripotent stem cells in a culture medium containing a PKC-β inhibitor and a BMP inhibitor to obtain an embryonic body (EB); and two-dimensionally differentiating the obtained embryonic body by two-dimensional culture to form a rosette structure. The pluripotent stem cells, neural precursor cells, differentiation, PKC-β inhibitor, BMP inhibitor, three-dimensional culture, and two-dimensional culture are as described above.

In an embodiment, the method may further include subculturing the cultured neural precursor cells.

According to another aspect of the disclosure, a use of neural precursor cells differentiated from pluripotent stem cells for preparation of a pharmaceutical composition for preventing or treating a degenerative brain disease is provided. In the use, the degenerative brain disease, prevention, treatment, pharmaceutical composition, pluripotent stem cells, differentiation, and neural precursor cells are as described above.

According to another aspect of the disclosure, a method of preventing or treating a degenerative brain disease comprising administering to an individual in need thereof an effective amount of neural precursor cells differentiated from pluripotent stem cells is provided. In the method, the pluripotent stem cells, differentiation, neural precursor cells, administration, degenerative brain disease, prevention, and treatment are as described above.

As used herein, the term "effective amount" may be easily determined by those skilled in the art based on factors well-known in the medical field such as the type of disease, age, body weight, health status, gender, and drug sensitivity of a patient, administration route, administration method, the number of administration, duration of treatment, and a drug used in combination or concurrently.

The individual may be mammals, such as humans, cows, horses, pigs, dogs, sheep, goats, or cats. The individual may be a subject in need of alleviating, preventive, or therapeutic effects of a degenerative brain disease, e.g., Alzheimer's disease.

### Advantageous Effects

By injecting the neural precursor cells differentiated from pluripotent stem cells to an amyloid beta-injected animal model through an intracerebroventricular route, the effect of strengthening cognitive function can be sustained for a long time, and thus, the neural precursor cells may be effectively used for treatment of degenerative brain diseases including Alzheimer's disease.

### Description of Drawings

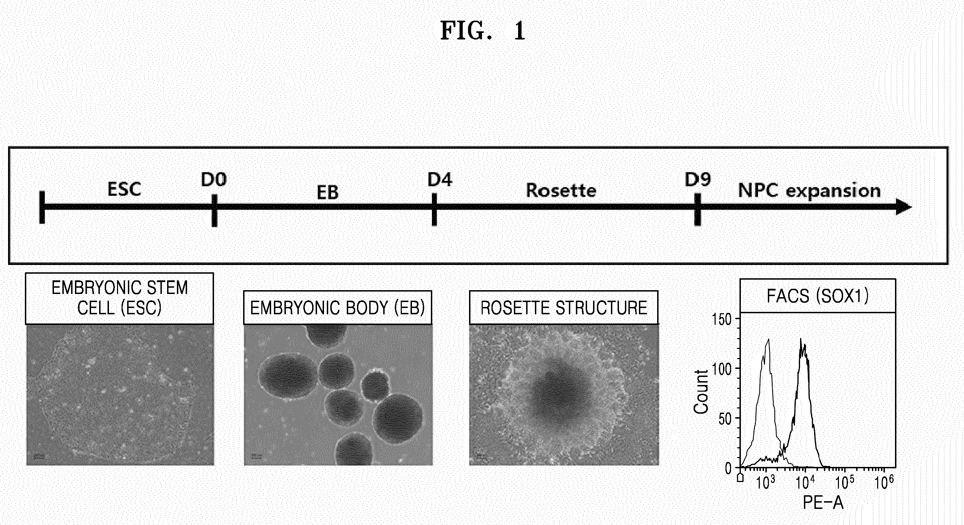

FIG. 1 is a diagram illustrating a differentiation method of NPCs and results of identifying expression of SOX.
FIG. 2 is a diagram illustrating a treatment schedule for an amyloid β injection animal model using NPCs according to an embodiment.
FIGS. 3A and 3B are graphs showing Y-maze evaluation results and NORT cognitive ability evaluation results of groups of an amyloid β--injected animal model administered with ES-MSCs via intravenous injection (IV), intrahippocampal injection (IH), and intracerebroventricular injection (ICV), respectively, on day 7, day 14, day 21, and day 28.
FIGS. 4A and 4B are graphs showing Y-maze evaluation results and NORT cognitive ability evaluation results of groups of an amyloid β-injected animal model administered with NPCs via intravenous injection (IV), intrahippocampal injection (IH), and intracerebroventricular injection (ICV), respectively, on day 7, day 14, day 21, and day 28.
FIGS. 5A and 5B are graphs showing Y-maze evaluation results and NORT cognitive ability evaluation results of groups of an amyloid β-injected animal model administered with NPCs of early passage (p2) and late passage (p12) via intracerebroventricular injection (ICV) on day 7, day 14, day 21, and day 28.
FIGS. 6A and 6B are graphs showing Y-maze evaluation results and NORT cognitive ability evaluation results of an amyloid β-injected animal model administered with NPCs via ICV injection observed for a long time of 6 weeks.

### Mode for Invention

Hereinafter, the present disclosure will be described in more detail with reference to the following examples. However, the following examples are merely presented to exemplify the present disclosure, and the scope of the present disclosure is not limited thereto.

### Example 1. Differentiation of Human Embryonic Stem Cell into Neural Precursor Cell

### 1.1. Culture of Human Embryonic Stem Cells

Human embryonic stem cells (hESCs) were cultured without feeder cells as follows.

Specifically, a 6-well tissue culture dish having a surface area of 9.6 cm² was treated with CTS Cellstart^{™}, Matrigel, Vitronectin, or Laminin, which is a cell adhesion enhancer, in a concentration of 1000 µ/well at 4 °C for 24 hours. Subsequently, after completely removing all of the remaining cell attachment enhancer from the tissue culture dish at room temperature, mTeSR^{™} (STEMCELL Technologies, CANADA), Essential 8^{™} (Gibco, USA), StemFit (AJINOMOTO, JAPAN), TeSR^{™}-E8^{™} (STEMCELL Technologies, CANADA) culture broth, a culture broth of embryonic stem cells, was added to the tissue culture dish at a concentration of 2000 µl/well, and then placed in an incubator at 37 °C under the condition of 5% CO₂.

Subsequently, human embryonic stem cells (CHA-hES NT 18, CHA university) proliferated on feeder cells were divided into small clumps by mechanical sub-passage using a Micro-tip (Axygen, USA), and the clumps were aliquoted into the tissue culture dish placed in the incubator at a density of 40 to 50 clumps/well. Then, the human embryonic stem cells were proliferated without the feeder cells while replacing the medium with a fresh embryonic stem cell conditioned medium (mTeSR^{™}) in a concentration of 500 µl/well every day for 5 days.

### 1.2. Induction of Differentiation of Human Embryonic Stem Cell into Neural Precursor Cell

An embryonic body (EB), which is a three-dimensional aggregate of pluripotent stem cells, was prepared from the human embryonic stem cells cultured under the feeder cell-free conditions according to Example 1.1, using collagenase. Under xenopathogen-free conditions, the cells were differentiated into neuroectoderms by three-dimensionally culturing for 4 days by adding a protein kinase C inhibitor (PKC-β inhibitor) and a BMP inhibitor (DMH1) thereto. Then, the EBs cultured in three dimensions were adhered to a cell culture dish and differentiated into two dimensions for 5 days to form a rosette structure having a certain shape and the rosette structure was physically obtained to isolate neural precursor cells (NPCs). To identify the NPCs as described above, expression of SRY-Box Transcription Factor 1 (SOX1), which is a transcription factor of a protein specific to neural precursor cells, was identified using a flow cytometry. Hereinafter, the neural precursor cell will be referred to as "NPC".

### Comparative Example 1. Differentiation of Human Embryonic Stem Cell into Mesenchymal Stem Cell

### 1.1. Induction of Differentiation of Human Embryonic Stem Cell into Ectodermal Cell

The human embryonic stem cells proliferated without feeder cells in Example 1.1 above were treated with a TGF-β inhibitor and a BMP inhibitor to be differentiated into ectodermal cells.

Specifically, the TGF-β inhibitor (SB431542) or the PKC-β Inhibitor and the BMP inhibitor (DMH1) were dissolved in dimethylsulfoxide (DMSO, Sigma, USA) in stock concentrations of 10 mM, 5 Mm, and 0.5 mM, respectively, and then diluted with an embryonic stem cell differentiation medium (DMEM/F12, 20 % (v/v) SR, 1 % (v/v) NEAA, 0.1 mM β-mercaptoethanol, 1 % (v/v) penicillin-streptomycin + SB341542 10 µM/ml+ 0.5 µM/ml or DMEM/F12, 20 % (v/v) SR, 1 % (v/v) NEAA, 0.1 mM β-mercaptoethanol, 1 % (v/v) penicillin-streptomycin+ PKC-β Inhibitor 5 µM/ml+ 0.5 µM/ml or DMEM/F12, Insulin 10 µM /ml, Transferrin 14 µM/ml, Selenium 9 µM/ml + SB341542 10 µM/ml or DMEM/F12, Insulin 10 µM /ml, Transferrin 14 µM/ml, and Selenium 9 µM/ml + PKC-β Inhibitor 5 µM/ml) to final concentrations of 10 µM, 5 µM, and 0.5 µM, respectively. Then, the human embryonic stem cells proliferated for 5 days in Example 1.1 were differentiated into ectodermal cells by treating the stem cells in the embryonic stem cell differentiation medium containing SB431542, the PKC-β Inhibitor, and DMH1 in a suspended state for 4 days.

### 1.2. Pretreatment of Ectodermal Cell

The ectodermal cells in the suspended state of Comparative Example 1.1 were treated with Matrigel and Vitronectin, which are cell adhesion enhancers, at room temperature for 1 hour. Then, all of the cell adhesion enhancers remaining on the tissue culture dish were removed at room temperature and the suspended cells were adhered thereto and cultured for 5 days in an ectodermal cell-inducing medium (DMEM/F12+ Insulin 25 µM/ml+ bFGF 20 µM/ml).

### 1.3. Induction of Differentiation into Mesenchymal Stem Cell

After identifying whether p75, which is a Neural crest marker, was expressed using a flow cytometry in Comparative Example 1.2 above, the ectodermal cells were differentiated using a mesenchymal stem cell medium ((DMEM/F12, 10 % (v/v) FBS, 4ng/ml bFGF, 1 % (v/v) NEAA, 0.1 mM β-mercaptoethanol, 1 % (v/v) penicillin-streptomycin or a-MEM, 10% (v/v) FBS, 4ng/ml bFGF or Cellartis^{®} MSC Xeno-Free Culture Medium or StemMACS^{™} MSC Expansion Media Kit or MSC NutriStem^{®} XF Medium or StemXVivo Xeno-Free Human MSC Expansion Medium or CellCor Serum Free Chemically Defined Medium), and subcultured to obtain mesodermal stem cells.

Specifically, all of the culture medium was removed on day 9 of differentiation, and then the cells were rinsed with PBS mixed with 1 % (v/v) penicillin-streptomycin. Then, the cells were treated with a StemPro^{™} Accutase^{™} cell dissociation reagent at 37 °C, under 5% CO₂ conditions for 5 minutes to prepare a single cell suspension and neutralized with a mesenchymal stem cell medium and then centrifuged at 1,000 rpm for 5 minutes. Subsequently, the cells obtained by centrifugation were aliquoted into 1 well/12 well plates previously coated with CTS Cellstart^{™}, Gelatin and continuously subcultured to a cell confluency of 1×10⁴ cells/cm² with a size in the order of 12 well plate (Passage 0) -> 6 well plate (Passage 1) -> T-25 flask (Passage 2) -> T-75 flask (Passage 3) whenever a cell confluency reached 80 to 90 %, by a method of preparing a single cell suspension by treatment with 0.05 % trypsin at room temperature. In this process, non-mesodermal stem cells were dropped, and mesodermal stem cells were obtained.

Hereinafter, the embryonic stem cell-derived mesenchymal stem cell will be referred to as "ES -MSC".

### Experimental Example 1. Identification of Therapeutic Effect of NPC on Dementia

### 1.1. Construction of Animal Model

6-week-old male B6 mice having a body weight of about 23 to 25 g were used and every 5 mice were housed in each cage under lighting for 12 hours at 21 °C and allowed free access to feed and water. Amyloid β₁₋₄₂ that is a substance causing memory loss was dissolved in 10% dimethyl sulfoxide (DMSO) saline and cultured at 37 °C for 1 week to a concentration of 100 µM. Each mouse was anesthetized by inhalation of 3 % isoflurane and 1.5 % isoflurane was maintained during a procedure using a respiratory anesthesia device. After preparing a stereotaxic device, a head of an experimental animal was fixed and injected with 5 µl of amyloid β₁₋₄₂ at a site 0.9 mm posterior, 1.7 lateral, and 2.2 in depth relative to the bregma using a 10 µl Hamilton microsyringe provided with a 26-gauge needle for 10 minutes.

### 1.2. Administration of NPC and Behavioral Evaluation

The NPCs prepared in Example 1 above at passage 3 obtained after subculturing three times and at passage 12 obtained after subculturing 12 times were used. Administration of the NPCs was conducted on day 7 after constructing the amyloid β-injected animal model, and all animal model experiments were conducted as blind tests. 3×10⁶ cells per vial were frozen, and the 3×10⁶ cells were dissolved in 300µl of a physiological saline and injected into each mouse via tail vein. 3×10⁶ cells were dissolved in 5 µl of the physiological saline and administered via intrahippocampal injection (IH) and intracerebroventricular injection (ICV) using a stereotaxic device, and the same amount of the physiological saline was administered to a control.

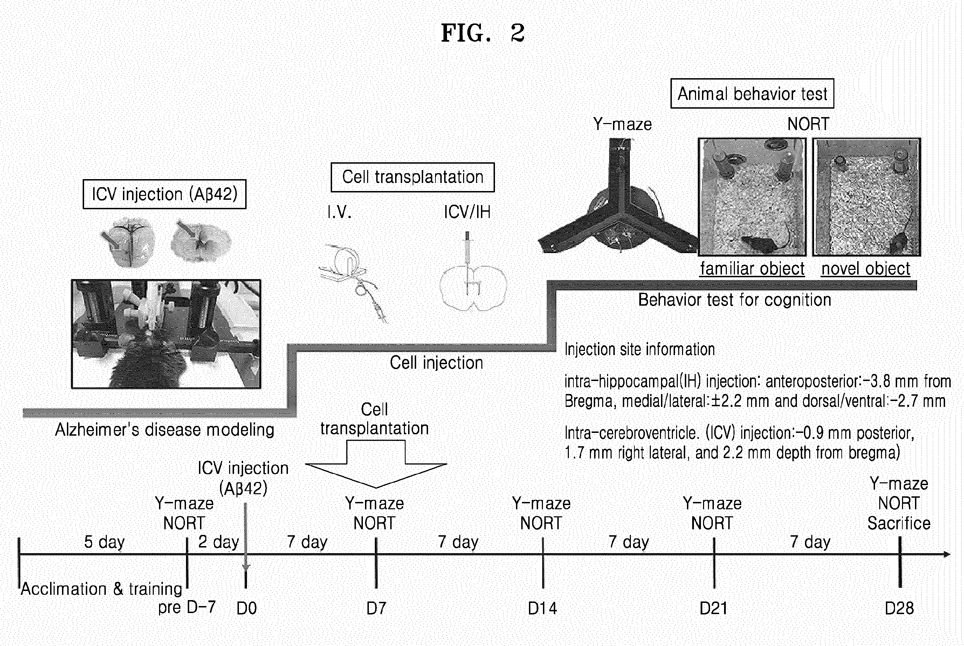

The mice were trained for Y-maze and NORT cognitive ability test before the procedure, and behavioral evaluation was performed by scoring the test results, and experiments were conducted by selecting lesion models on day 5 after the procedure. FIG. 2 is a diagram illustrating a treatment schedule for an amyloid β injection animal model using NPCs according to an embodiment. After 5 days from treatment, the mice were treated with cell lines and evaluated on day 7, day 14, day 21, and day 28 whether the cognitive function is enhanced.

Behavioral evaluation was performed by conducting Y-maze test and NORT cognitive ability evaluation.

Y-maze test: A device including 3 passages (arms) and shaped like the letter Y was used. Each maze arm has a length of 35 cm, a height of 15 cm, and a width of 5 cm and is located at the same angle. Movement of an experimental animal is represented by the number of crossovers, and consecutive triplets of different arm choices is defined as one crossover (spontaneous alteration). Each experimental animal was allowed to start from one maze arm and move to another maze arm, and the number of successful crossover is scored and expressed as a percentage. The mice were allowed to move freely for 2 minutes and then measured for 5 minutes.

Novel object recognition test (NORT): A method for testing cognitive ability by stimulating curiosity of a rodent. After adapting an animal with two identical objects during an adaptation period, the memory of the animal is measured by inducing innate curiosity by replacing one of the familiar objects with a novel object, i.e., an object with different color. The mice were allowed to move freely to familiar objects for 2 minutes. After changing one object with a novel object, the behavior was observed for 5 minutes. A period spent on the novel object divided by the sum of periods spent on each object was expressed as a percentage.

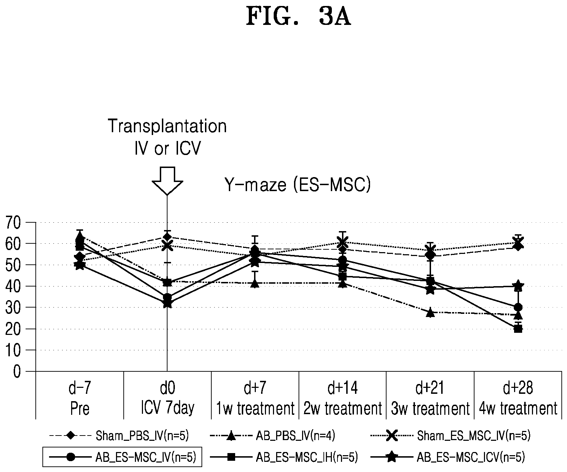

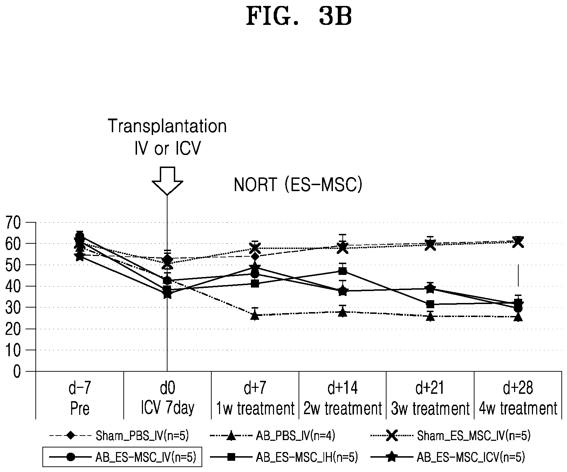

FIGS. 3A and 3B are graphs showing Y-maze evaluation results and NORT cognitive ability evaluation results of groups of an amyloid β-injected animal model administered with ES-MSCs via intravenous injection (IV), intrahippocampal injection (IH), and intracerebroventricular injection (ICV), respectively, on day 7, day 14, day 21, and day 28.

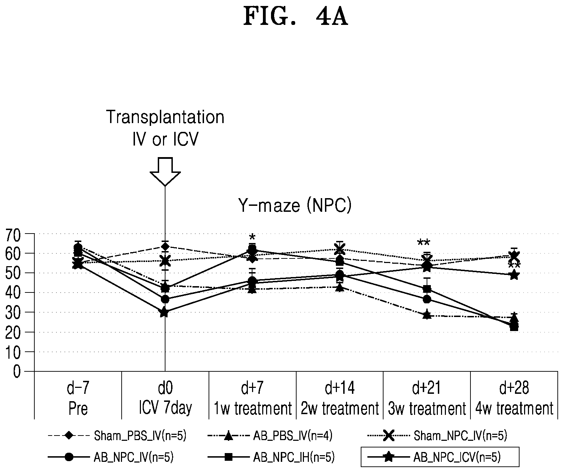

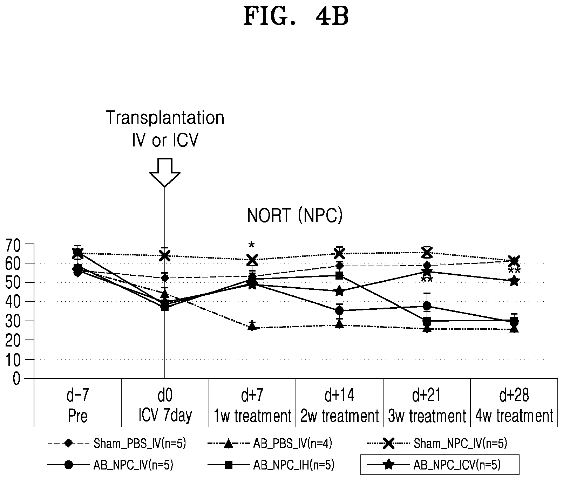

FIGS. 4A and 4B are graphs showing Y-maze evaluation results and NORT cognitive ability evaluation results of groups of an amyloid β-injected animal model administered with NPCs via intravenous injection (IV), intrahippocampal injection (IH), and intracerebroventricular injection (ICV), respectively, on day 7, day 14, day 21, and day 28.

As a result, as shown in FIGS. 3A and 3B, when the ES-MSCs were administered on day 7 after the preparation of the amyloid β-injected animal model, it was confirmed that the IV injection exhibited efficacy and the efficacy decreased from week 3, and the other injection methods exhibited increases in efficacy within 1 to 2 weeks, but the efficacy tended to decrease thereafter. Unlike this, in the case of the NPCs, as shown in FIGS. 4A and 4B, when the NPCs were administered on day 7 after the preparation of the amyloid β-injected animal model, both IV and IH injections seemed to show cognitive function strengthening effects for 2 weeks, but the effects decreased, whereas ICV injection exhibited that the cognitive function strengthening effect was maintained for 4 weeks.

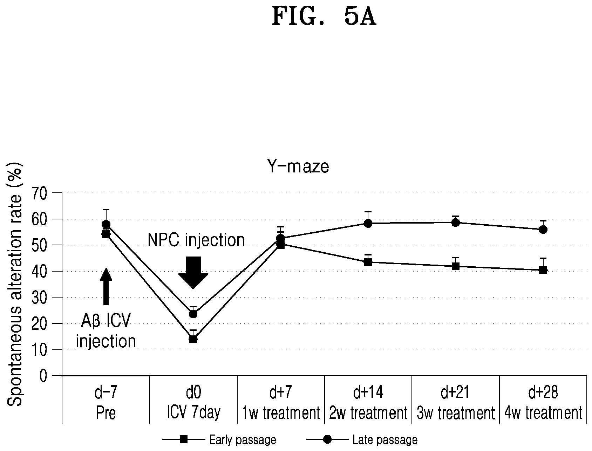

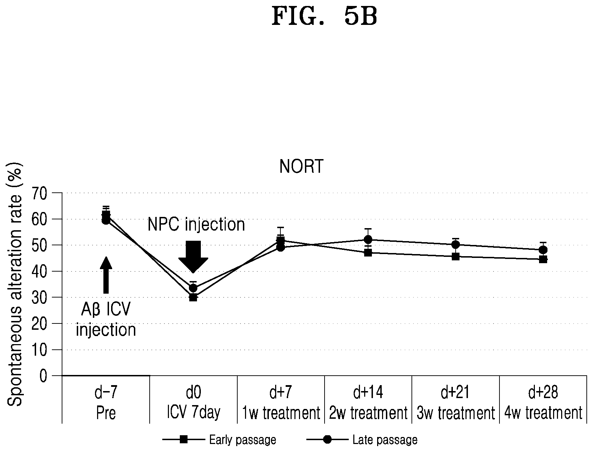

FIGS. 5A and 5B are graphs showing Y-maze evaluation results and NORT cognitive ability evaluation results of groups of an amyloid β-injected animal model administered with NPCs of early passage (p2) and late passage (p12) via intracerebroventricular injection (ICV) on day 7, day 14, day 21, and day 28.

As a result, as shown in FIGS. 5A and 5B, it was confirmed that cognitive function strengthening effects were observed in both groups in which NPCs cultured until the early passage (p2) and the late passage (p12) were administered via ICV injection.

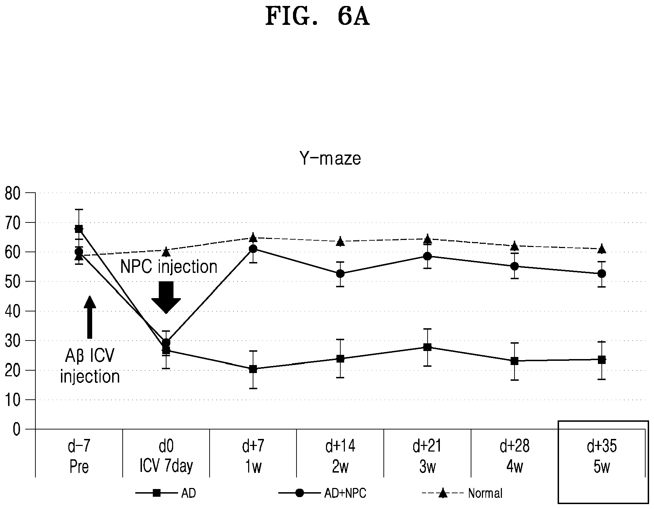

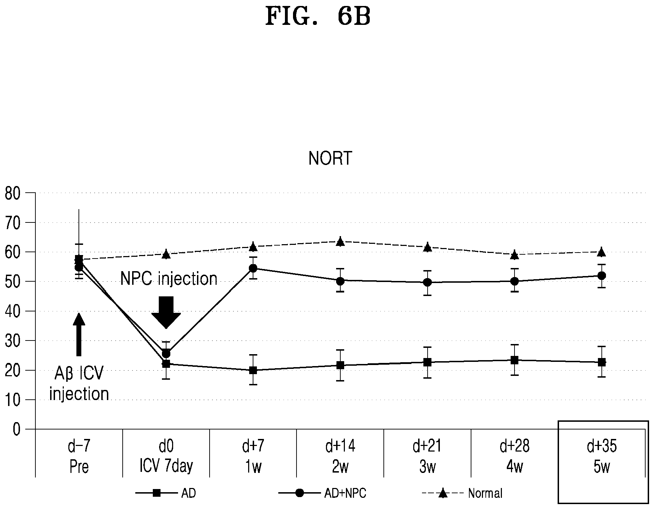

FIGS. 6A and 6B are graphs showing Y-maze evaluation results and NORT cognitive ability evaluation results of an amyloid β-injected animal model administered with NPCs via ICV injection observed for a long time of 6 weeks.

As a result, referring to FIGS. 6A and 6B, it was confirmed that the cognitive function was strengthened in the group administered with NPCs via intracerebroventricular (ICV) injection to a level similar to that of the normal group, compared to the group not administered with the NPCs after injection of amyloid β. It was also confirmed that the cognitive function strengthening effects were prolonged for a long period of time of 6 weeks.

In conclusion, NPCs have superior effects on prolonging the cognitive function strengthening effects to ES-MSCs. Particularly, in the case of administering the NPCs via intracerebroventricular (ICV) injection, the cognitive function strengthening effects may be maintained for a long period of time compared to other administration routes, indicating that significant therapeutic effects may be obtained thereby in degenerative brain diseases such as Alzheimer's disease.

## Claims

1. A pharmaceutical composition for preventing or treating a degenerative brain disease comprising neural precursor cells differentiated from pluripotent stem cells as an active ingredient.

2. The pharmaceutical composition of claim 1, wherein the pluripotent stem cells are selected from the group consisting of nuclear transfer pluripotent stem cells (NT-hPSCs), parthenote-derived pluripotent stem cells (pn-hPSCs), induced pluripotent stem cells (iPSCs), and embryonic stem cells (ESCs).

3. The pharmaceutical composition of claim 1, wherein the degenerative brain disease is a disease selected from the group consisting of Alzheimer's disease, Parkinson's disease, Huntington's disease, mild cognitive impairment, cerebral amyloid angiopathy, Down's syndrome, amyloidotic stroke, systematic amyloidosis, Dutch-type amyloidosis, Niemann-Pick disease, senile dementia, amyotrophic lateral sclerosis, spinocerebellar atrophy, Tourette's syndrome, Friedrich's Ataxia, Machado-Joseph's disease, Lewy body dementia, dystonia, progressive supranuclear palsy, and frontotemporal dementia.

4. The pharmaceutical composition of claim 1, wherein the composition is administered via an intravenous (IV), intrahippocampal (IH), intracerebral, intracranial, intraspinal, intracerebrospinal, intracerebroventricular (ICV), or intrathecal route.

5. The pharmaceutical composition of claim 1, wherein the neural precursor cells are differentiated by two-dimensionally culturing an embryonic body (EB) obtained by three-dimensionally culturing the pluripotent stem cells in a culture medium including a protein kinase C-β (PKC-β) inhibitor and a bone morphogenetic protein (BMP) inhibitor.

6. The pharmaceutical composition of claim 5, wherein the pluripotent stem cells are obtained by culturing under feeder cell-free conditions.

7. The pharmaceutical composition of claim 5, wherein the PKC-β inhibitor is selected from: 2-[1-(3-dimethylaminopropyl)-5-methoxy indol-3-yl]-3-(1H-indol-3-yl) maleimide; 3-(1-(3-imidazol-1-yl propyl)-1H-indol-3-yl)-4-anilino-1H-pyrrole-2,5-dione; 3-(1H-indol-3-yl)-4-[2-(4-methylpiperazin-1-yl)quinazolin-4-yl]pyrrole-2,5-dione; (3-{1-[3-(amidinothio)propyl]-1H-indol-3-yl}-3-(1-methyl-1H-indol-3-yl)maleimide methane sulfonate; 13-hydroxyoctadecadienoic acid; bisindolylmaleimide; 2,6-diamino-N-([1-oxotridecyl)-2-piperidinyl]methyl)hexanamide; 4'-demethylamino-4'-hydroxystaurosporine; and 3-(13-methyl-5-oxo-6,7-dihydro-5H-indolo[2,3-a]pyrrolo[3,4-c]carbazol-12(13H)-yl)propanenitrile.

8. The pharmaceutical composition of claim 5, wherein the BMP inhibitor is selected from: dorsomorphin (6-[4-[2-(1-piperinyl)ethoxy]phenyl]-3-(4-pyridinyl)-pyrazolo[1,5-a]pyrimidine)); dorsomorphin homolog 1 (DMH1, 4-[6-[4-(1-methylethoxy)phenyl]pyrazolo[1,5-a]pyrimidine-3-yl]-quinoline); K 02288 (3-[(6-amino-5-(3,4,5-trimethoxyphenyl)-3-pyridinyl]phenol); LDN 212854 (5-(6-(4-(1-piperazinyl)phenyl)pyrazolo[1,5-a]pyrimidine-3-yl)quinolone); and Noggin polypeptide.

9. The pharmaceutical composition of claim 5, wherein the three-dimensionally culturing is performed for 1 day to 10 days.

10. The pharmaceutical composition of claim 5, wherein the two-dimensionally culturing is performed for 1 day to 15 days.

11. A use of neural precursor cells differentiated from pluripotent stem cells for preparation of a pharmaceutical composition for preventing or treating a degenerative brain disease.

12. A method of preventing or treating a degenerative brain disease, the method comprising: administering to an individual in need thereof an effective amount of neural precursor cells differentiated from pluripotent stem cells.
